# EUROPEAN PATENT APPLICATION

(11) **EP 1 762 307 A1**
(43) Date of publication of application: **14.03.2007**
(21) Application number: 05753449.7
(22) Date of filing: 22.06.2005
(51) Int. Cl.: B08B 3/12, A61L 2/18, B06B 1/00

(54) **ULTRASONIC CLEANING METHOD AND APPARATUS**

(30) Priority: 29.06.2004 JP 2004191999
(71) Applicant: Kagoshima Supersonic Technical Laboratoy Co., Ltd., Kagashima-shi Kagoshima, 8900045 (JP)
(72) Inventor: SHIGIHARA, Takanori, EROICA CORPORATION, Ashiya-shi Hyogo, 6590065 (JP); UEDA, Toyotoshi, 1930944 (JP)
(74) Representative: Sherrard-Smith, Hugh
(86) International application number: PCT/JP2005/011415
(87) International publication number: WO 2006/001293

(57) **Abstract**

An ultrasonic cleaning method and an ultrasonic cleaning device are provided, which are capable of performing simultaneously cleaning and sterilization of medical appliances or the like, and which can also be used for washing hands for disinfection without any special waste water treatment. The ultrasonic cleaning method for cleaning an object to be cleaned by using an ultrasonic wave includes the steps of putting cleaning fluid and the object to be cleaned in a cleaning tank (16), and applying ultrasonic vibrations to the cleaning fluid from an ultrasonic vibration member disposed in the cleaning tank (16) while ejecting air containing ozone into the cleaning fluid. The vibration portion has a communicating conduit 40 that opens at the surface thereof, and air containing ozone is discharged into the cleaning fluid from the communicating conduit. A silver electrode is disposed in the cleaning fluid, and electrolytic sterilization by silver ions is performed for the object to be cleaned adding to sterilization by ozone.

## Description

### TECHNICAL FIELD

The present invention relates to an ultrasonic cleaning method and an ultrasonic cleaning device for cleaning an object by applying ultrasonic vibration to a cleaning fluid inside a cleaning tank.

### BACKGROUND ART

Conventionally, medical appliances such as scissors and forceps that were used for an operation or a therapy in a hospital or the like are cleaned or sterilized by the following methods.
(1) Removal of blood or the like by using hot water or an enzyme treatment (hereinafter referred to as a "precleaning").
(2) Ultrasonic cleaning by using a detergent.
(3) Sterilization by using an autoclave with high-pressure steam at a temperature of 135 degrees centigrade, gas sterilization by using ethylene oxide gas, or sterilization by dipping in 2% glutaraldehyde solution.

In addition, clothes such as a white garment are sterilized by using high-pressure steam after a usual laundry for removing dirty things.

The devices as mentioned above in (2), which clean objects by applying ultrasonic vibrations, are available on the market at present. However, the objects that can be cleaned by the ultrasonic cleaning are limited to things having a high mechanical impedance (also referred to as "specific acoustic impedance", which is expressed by density x propagation rate), e.g., glasses, jewelry or metallic things, so fabrics such as clothes or gauze, leathers or other soft and flexible things cannot be cleaned by the ultrasonic cleaning. An ultrasonic cleaning device disclosed in Japanese unexamined utility model publication No. 63-73187 is aimed at solving the problem in the ultrasonic cleaning described above. The device sends air bubbles into the cleaning fluid and applies the ultrasonic vibration to the cleaning fluid simultaneously, so that dirty things sticking to the object to be cleaned are removed by a synergistic effect of the ultrasonic energy and the air bubbles.

### DISCLOSURE OF THE INVENTION

The conventional method for cleaning and sterilizing medical appliances needs three process steps including the precleaning step using the enzyme treatment, the ultrasonic cleaning step and the sterilization step, and its process is complicated. In addition, the sterilization method by using the high-pressure steam cannot be used for an endoscope or other appliance that is not suitable for high temperature processing. The gas sterilization method must use the ethylene oxide that is explosive and dangerous. The sterilization method using the glutaraldehyde also has a problem that the glutaraldehyde has chronic toxicity.

Furthermore, in a hospital or other places, it is a practice to wash hands before an operation or accessing a room or the like. Recently in particular, hospital infections caused by MRSA (Methicillin Resistant Staphylococcus Aureus) have been a serious situation, so importance of washing hands has been enhanced for health professionals and visitors. As a disinfectant for washing hands, a cationic detergent such as benzalkonium chloride is generally used.

Medical waste water ejected from a large scale hospital or the like in a quantity over an emission regulation must be processed individually before being discharged to a public sewerage or a river because it contains a lot of organic matters that increase BOD (Biochemical Oxygen Demand). In many cases, a process of the medical waste water uses a method of decreasing the BOD with an activated sludge facility for decomposing organic matters such as a detergent by using microorganisms. However, if waste water of washing hands that has a bactericidal effect is mixed a lot into the medical waste water to be decomposed by the microorganisms, it may cause a problem of perishing the microorganisms in the activated sludge.

In view of the above-mentioned problems, an object of the present invention is to provide an ultrasonic cleaning method and an ultrasonic cleaning device that can clean and sterilize medical appliances or the like simultaneously. The method and the device should also be used for washing hands in the purpose of disinfection. In addition, the method and the device should not need any special process when ejecting waste water.

### BRIEF DESCRIPTION OF THE DRAWINGS

Fig. 1 is a diagram showing a structure of an ultrasonic cleaning device according to a first embodiment of the present invention.
Fig. 2 is a diagram showing a structure of an ozone generation device.
Fig. 3 is a front cross section of an ultrasonic vibration transducer.
Fig. 4 is a plan view of the ultrasonic vibration transducer.
Fig. 5 is a perspective view showing the ultrasonic vibration transducer in an exploded manner.
Fig. 6 is a perspective view of the ultrasonic cleaning device.
Fig. 7 is a diagram showing an arrangement of the ultrasonic vibration transducers in the ultrasonic cleaning device.
Fig. 8 is a side cross section of a cleaning tank of an ultrasonic cleaning device according to a second embodiment of the present invention.
Fig. 9 is a front cross section of the ultrasonic cleaning device shown in Fig. 8.
Fig. 10 is a side cross section of a cleaning tank of an ultrasonic cleaning device according to a third embodiment of the present invention.
Fig. 11 is a front cross section of the ultrasonic cleaning device shown in Fig. 10.
Fig. 12 is a cross section of a cleaning tank of an ultrasonic cleaning device according to a fourth embodiment of the present invention.
Fig. 13 is a front view of the electrode plate.
Fig. 14 is a cross section of a cleaning tank of an ultrasonic cleaning device according to a fifth embodiment of the present invention.
Fig. 15 is a cross section of an ultrasonic cleaning device according to a first variation.
Fig. 16 is a cross section of an ultrasonic cleaning device according to a second variation.
Fig. 17 is a perspective view of an ultrasonic cleaning device with a lid opened according to a third variation.
Fig. 18 is a perspective view of an ultrasonic cleaning device with the lid closed according to the third variation.

### BEST MODE FOR CARRYING OUT THE INVENTION

### [First embodiment]

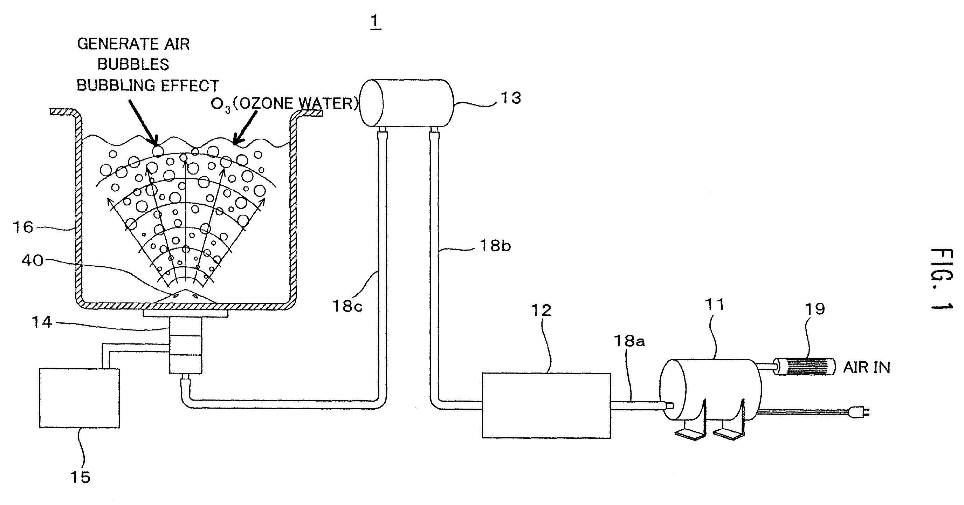

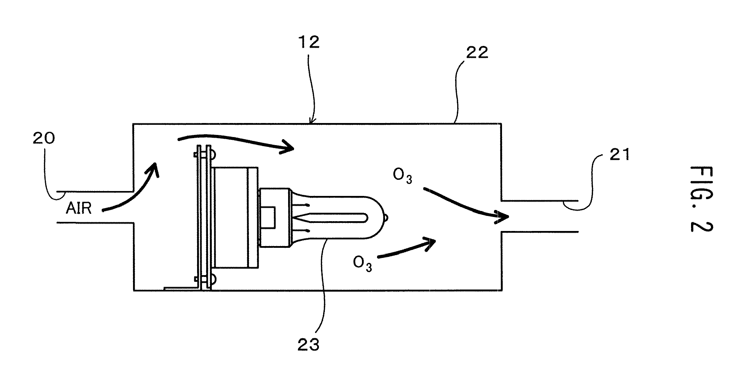

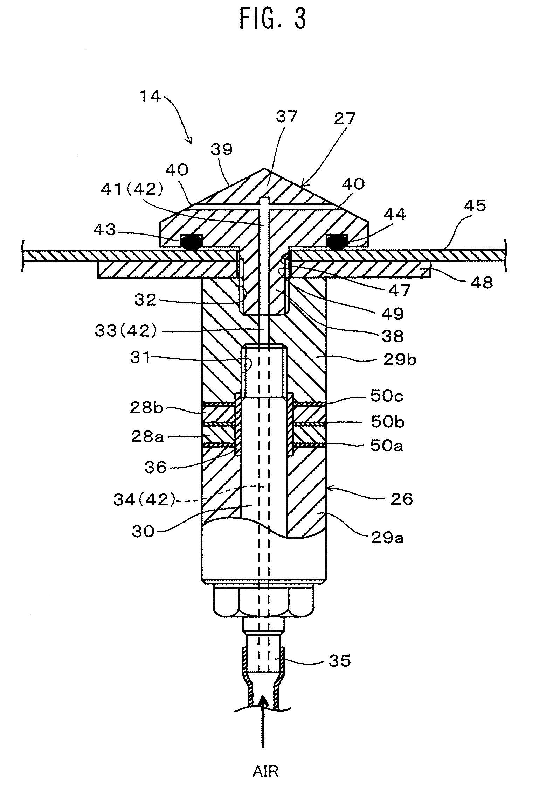

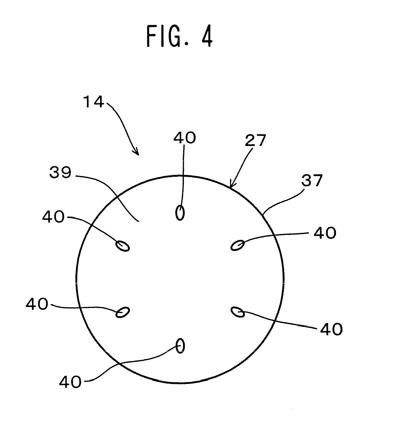

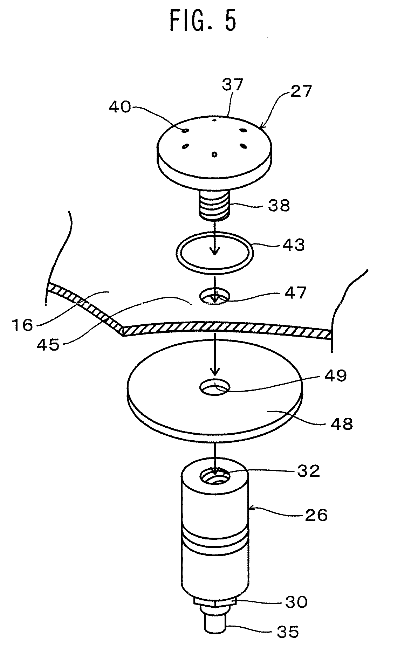

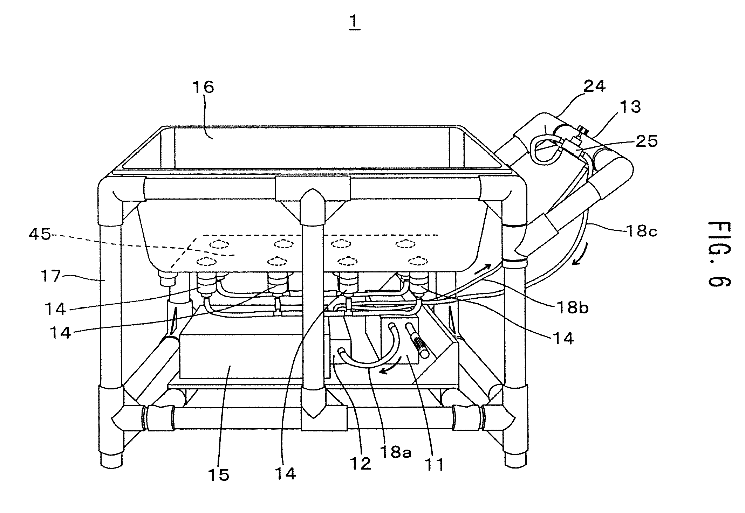

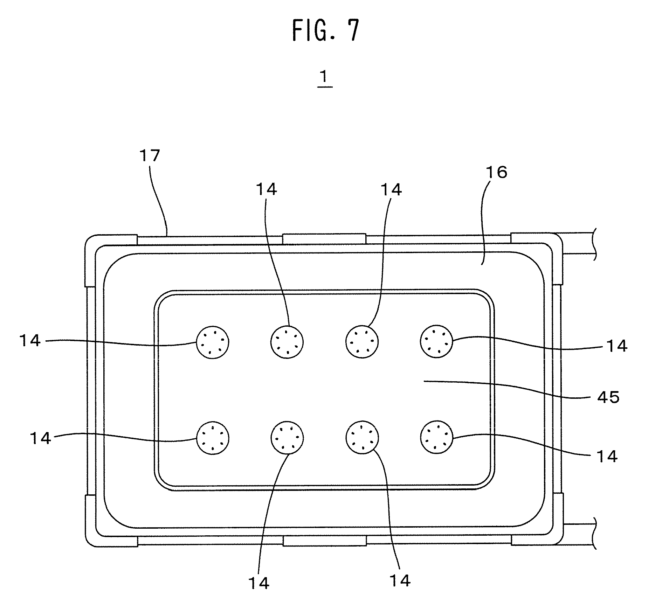

Fig. 1 is a diagram showing a structure of an ultrasonic cleaning device 1 according to a first embodiment of the present invention, Fig. 2 is a diagram showing a structure of an ozone generation device 12, Fig. 3 is a front cross section of an ultrasonic vibration transducer 14, Fig. 4 is a plan view of the ultrasonic vibration transducer 14, Fig. 5 is a perspective view showing the ultrasonic vibration transducer 14 in an exploded manner, Fig. 6 is a perspective view of the ultrasonic cleaning device 1, and Fig. 7 is a diagram showing an arrangement of the ultrasonic vibration transducers 14 in the ultrasonic cleaning device 1.

In Figs. 1-7, the ultrasonic cleaning device 1 is made up of a compressor 11, the ozone generation device 12, a buffer tank 13, an ultrasonic transducer 14, a drive portion (a drive circuit) 15 and a cleaning tank 16. As shown in Fig. 6, the compressor 11, the ozone generation device 12, the buffer tank 13, the ultrasonic transducer 14, the drive portion 15 and the cleaning tank 16 are attached to a pedestal 17 constructed with tubular members made of resin, and they are integrated. The compressor 11, the ozone generation device 12, the buffer tank 13 and the ultrasonic transducer 14 are connected to each other via silicone tubes 18a-18c having resistance to ozone for a special medical use so that air is supplied to them.

The compressor 11 takes air in and compresses the air, which is sent to the ozone generation device 12. The compressed air is sent to the inside of the ultrasonic transducer 14 via the ozone generation device 12 and the buffer tank 13, and the air is discharged into the inside of the cleaning tank 16 through injection holes 40 provided to a vibration portion 27 of the ultrasonic transducer 14. An intake side of the compressor 11 is provided with a filter 19 for removing dust. A diaphragm pump is used for the compressor 11.

The ozone generation device 12 is used for generating ozone from the air supplied from the compressor 11. As shown in Fig. 2, the ozone generation device 12 includes an ozone lamp 23 housed in a case 22 with an air inlet 20 and an ozone outlet 21. A UV lamp that emits ultraviolet rays is used as the ozone lamp 23. The air ejected from the compressor 11 goes to the inside of the ozone generation device 12 through the air inlet 20 and flows inside the ozone generation device 12 while it is exposed to the ultraviolet rays emitted from the ozone lamp 23. Thus, a part of oxygen is converted into ozone due to irradiation of the ultraviolet rays. The air containing ozone is sent to the buffer tank 13 from the ozone outlet 21. The case 22 is made of a material such as a metal that is not deteriorated by the ultraviolet rays.

The buffer tank 13 is used for reducing a ripple of the air ejected from the compressor 11 (a diaphragm pump). The ultrasonic cleaning device 1 has a handle 24 of the pedestal 17, and a space inside the handle 24 is divided for making the buffer tank 13. The air containing the ozone is sent from the buffer tank 13 to the ultrasonic transducer 14 via a regulating valve 25. The regulating valve 25 is fixed to the handle 24 and regulates a flow rate of the air containing the ozone discharged into the cleaning tank 16.

The ultrasonic transducer 14 is attached to a bottom portion 45 of the cleaning tank 16 and applies ultrasonic vibration to cleaning fluid in the cleaning tank 16 and an object to be cleaned. The ultrasonic transducer 14 ejects also the air containing the ozone into the cleaning fluid.

As shown in Fig. 3, the ultrasonic transducer 14 is a bolting oscillator including an oscillator 26 and the vibration portion 27 that are fastened to each other.

The oscillator 26 is made up of two ring-like electrostriction piezoelectric ceramic elements 28a and 28b arranged in the axial direction and two block members 29a and 29b that sandwich the piezoelectric ceramic elements 28a and 28b. The piezoelectric ceramic elements 28a and 28b and the block members 29a and 29b are integrated by a connecting bolt 30, which penetrates one block member 29a along its axis and engages an internal thread 31 formed at an end portion of the other block member 29b to be fastened. The other end of the block member 29b is provided with another internal thread 32 for being connected to the vibration portion 27. Two holes of the two internal threads 31 and 32 of the block member 29b communicate with each other via an air hole 33. The connecting bolt 30 has a through hole 34 that passes through it along its axis. When the connecting bolt 30 is screwed to the internal thread 31 of the block member 29b, the through hole 34 communicates with the air hole 33 of the block member 29b. The head portion side of the connecting bolt 30 is provided with an opening of the through hole 34 and a connecting portion 35 that is inserted into a silicone tube or the like.

Two piezoelectric ceramic elements 28a and 28b are arranged so as to have opposite polarities to each other. Total three ring-like electrode plates 50a-50c are arranged. One electrode is placed between them, and two electrodes sandwich them from both sides. The electrode plates 50a and 50c on both sides are connected electrically to each other and are applied with a common potential. The electrode plates 50a and 50c and the electrode plate 50b are connected to a drive circuit of the drive portion 15. In addition, an insulator ring 36 is provided between the connecting bolt 30 and the electrode plates 50a-50c so that the three electrode plates 50a-50c are insulated from each other.

As shown in Figs. 3 and 4, the vibration portion 27 includes a head portion 37 and an external thread portion 38. The head portion 37 has substantially a conical shape, and its axis is common with the axis of the external thread portion 38. The vibration portion 27 is provided with an air passage 41 that passes along the axis of the external thread portion 38 and diverges into six directions that are perpendicular to the axis inside the head portion 37. Each branch of the diverging air passage 41 has an injection hole 40 that opens on a tapered surface 39 of the head portion 37. The air passage 41 forms a path 42 from the connecting portion 35 to the injection holes 40 together with the air hole 33 of the block member 29b and the through hole 34 of the connecting bolt 30 when the external thread portion 38 is engaged with the internal thread 32. The lower surface of the head portion 37 is provided with a circular groove 44 in which an O ring 43 fits. When the ultrasonic transducer 14 is attached to the cleaning tank 16, the vibration portion 27 contacts the bottom portion 45 of the cleaning tank 16 via the O-ring 43 fitting in the groove 44. Therefore, propagation of the ultrasonic vibration to the bottom portion 45 from the vibration portion 27 is weakened, so that a generation of erosion on the bottom portion 45 can be prevented.

It will be described later that the ultrasonic transducer 14 can be attached to a side wall of the cleaning tank 16. In this case, the O-ring 43 fitting in the groove 44 prevents a generation of the erosion on the side wall. Instead of the O ring a rubber gasket or a gasket made of an organic fiber or an inorganic fiber can be used.

As shown in Figs. 3 and 5, the ultrasonic transducer 14 is fixed to the cleaning tank 16 by inserting the external thread portion 38 in an attachment hole 47 provided to the bottom portion 45 of the cleaning tank 16, and by engaging and fastening the external thread portion 38 with the internal thread 32 of the block member 29b. When this fixing is performed, an auxiliary plate 48 is sandwiched between the bottom portion 45 of the cleaning tank 16 and the oscillator 26. The auxiliary plate 48 is a disk-like metal plate having an outer diameter substantially three times larger than an outer diameter of the oscillator 26. A through hole 49 having a diameter that is substantially equal to a diameter of the attachment hole 47 is formed at the center of the auxiliary plate 48. It is possible to attach the ultrasonic transducer 14 to the cleaning tank 16 without using the auxiliary plate 48.

As shown in Figs. 6 and 7, the ultrasonic cleaning device 1 has total eight of the ultrasonic transducers 14 that are arranged on the bottom portion 45 of the cleaning tank 16 in two parallel lines at a constant pitch among four on each line. Each of the ultrasonic transducers 14 provided to the ultrasonic cleaning device 1 has an output power of 50 watts or less. The regulating valve 25 is connected to the silicone tube 18c, which diverges into two below the cleaning tank 16, and each of the two further diverges to be connected to the connecting portion 35 of the ultrasonic transducer 14 of each line.

The drive portion 15 incorporates a drive circuit for driving the piezoelectric ceramic elements 28a and 28b of each ultrasonic transducer 14 to generate ultrasonic vibrations. The drive circuit is provided individually for each ultrasonic transducer 14. Since each of the ultrasonic transducers 14 has the individual drive circuit so that an output power of every ultrasonic transducer 14 is regulated below 50 watts, unwanted electromagnetic radiations or line noises due to the ultrasonic cleaning device 1 can be reduced. For example, even if the ultrasonic cleaning device 1 is used in a hospital or a research laboratory for cleaning medical appliances and clothes or for washing hands, it is possible to avoid a problem of "unwanted electromagnetic radiation noises (spurious radiation)" that may cause various abnormal operations in computers, many electronic monitors, operating devices, pace makers and the like. This method uses a set of individual electric drives and a set of ultrasonic oscillators having a power of 50 watts at most. Therefore, it is driven by a small electric power and is advantageous to the above-mentioned problem. In addition, it is equipped with filters of "common canceling" provided to each of them individually.

The cleaning tank 16 is made of stainless steel and fixed to the pedestal 17 via a rubber sheet (not shown). The rubber sheet has a function of stopping a vibration of the cleaning tank 16 so that the pedestal 17 is not broken down. A fabric tape is wound around a circumference surface (not shown) of the cleaning tank 16. The fabric tape absorbs the ultrasonic vibration of the circumference surface of the cleaning tank 16 by its elasticity and reduces noises that may be generated on the circumference surface of the cleaning tank 16. Instead of using the fabric tape it is possible to coat the circumference surface of the cleaning tank 16 with gum resin, or other material having elasticity for obtaining the same effect.

Next, a cleaning process by the ultrasonic cleaning device 1 will be described.

The cleaning process uses water as the cleaning fluid. The cleaning process includes steps of placing an object to be cleaned such as a medical instrument including an endoscope and an operation instrument or clothes including a white garment and the like in the cleaning tank 16, and applying ultrasonic vibrations to the object from the ultrasonic transducer 14 while ejecting air including ozone from the injection holes 40 of the vibration portion 27 into the cleaning tank 16.

The ultrasonic transducer 14 outputs the ultrasonic vibrations at frequencies of 25-100 kHz usually. Within this frequency range, the frequency is selected in accordance with a material, strength and the like of the object to be cleaned. In general, a high frequency is preferable if the object to be cleaned has a fine structure with micro spaces. It is possible to use a lower or a higher frequency than the above-mentioned frequency range as the frequency of the ultrasonic vibration.

The air that is ejected into the water inside the cleaning tank 16 through the ozone generation device 12 contains ozone at a ratio within a range from a few ppm to a few tens ppm or to a few hundred ppm. The higher a concentration of the ozone is, the higher the effect of the cleaning and sterilization. If the cleaning process is performed in an unattended room that is equipped with a ventilation device or an exhaust device, the ozone concentration in the air ejected from the injection holes 40 can be raised. Since the ozone has toxicity, however, it is preferable to lower the ozone concentration if the cleaning process is performed in a room where someone always exists. The ozone concentration in the air supplied from the ozone generation device 12 is affected by a time period (residence time) while the air in the ozone generation device 12 is irradiated with the ultraviolet rays from the ozone lamp 23 or other factors. For example, increasing the ozone concentration can be realized by raising the output power of the ozone lamp or by reducing a quantity of the air flowing through the ozone generation device 12. Alternatively, in the designing stage, the case 22 of the ozone generation device 12 is arranged to be large so that the residence time of the air inside the case becomes long. Note that electric discharge may be used instead of irradiation of the ultraviolet rays for generating ozone.

In the cleaning process performed by the ultrasonic cleaning device 1, bubbles of the air ejected from the injection holes 40 rise and disturb the liquid surface, so the depth of the liquid varies continuously. This continuous variation of the liquid depth causes continuous variation of sound pressure distribution of a standing wave that is generated by the ultrasonic vibration radiated from the vibration portion 27 and the ultrasonic vibration reflected by the liquid surface. Thus, the inside of the cleaning tank 16 becomes apparently a state as if there is a sound pressure distribution of a traveling wave. Therefore, the ultrasonic cleaning device 1 of this embodiment can obtain a synergistic effect of the ultrasonic energy and micro bubbles due to the radiation of the traveling wave, so that even a soft object can be cleaned effectively. In addition, there is little cleaning spots, i.e., uneven cleaning due to the standing wave that was a problem in the conventional ultrasonic cleaning.

In addition, the bubbles of the air ejected from the injection holes 40 generate a circulating flow inside the cleaning tank 16 accompanied by the surrounding water when the bubbles go up (jet stream effect). The bubbles are partially shattered to be micro bubbles by the ultrasonic wave, and its speed of rising is decreased so they easily accompany the circulating flow to reach the surface of the fine structure of the object to be cleaned. The micro bubbles increase substantially a contact area that is an area of the interface between the ozone in the bubbles and the water so as to promote dissolution of the ozone and a reaction between the ozone and the water. The reaction between the ozone and the water generates hydroxy radical that has a strong oxidation ability and sterilization ability. The hydroxy radical decomposes dirt such as blood or sebum sticking to the object to be cleaned and deadens harmful bacteria lurking in the dirt by its strong oxidation ability. Thus, the ultrasonic cleaning device 1 performs the cleaning of the object to be cleaned and the sterilization of the same simultaneously.

In the cleaning process by the ultrasonic cleaning device 1, organic matters that are main components of the dirt are decomposed almost completely by the hydroxy radical into carbon dioxide and water. If the object to be cleaned is clothes, not only deodorizing but also bleaching is performed. Waste water after the cleaning process contains little organic matter that increases the BOD value, and the ozone and the hydroxy radical in the water is transformed into oxygen or water in a short time period. Therefore, the waste water after the cleaning process can be discharged into the sewerage without a special waste water treatment while suppressing a secondary pollution to environment.

If the ultrasonic cleaning device 1 is used for sterilization such as washing hands in a school or in a hospital for accessing a room, it is preferable to set the ozone concentration in the air ejected into the water to a low value and to install the ultrasonic cleaning device 1 in a place with a good ventilation in order to prevent the ozone from causing bad influence to health.

### [Second embodiment]

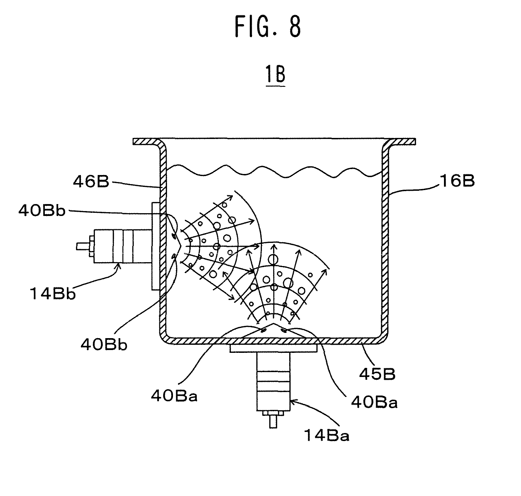

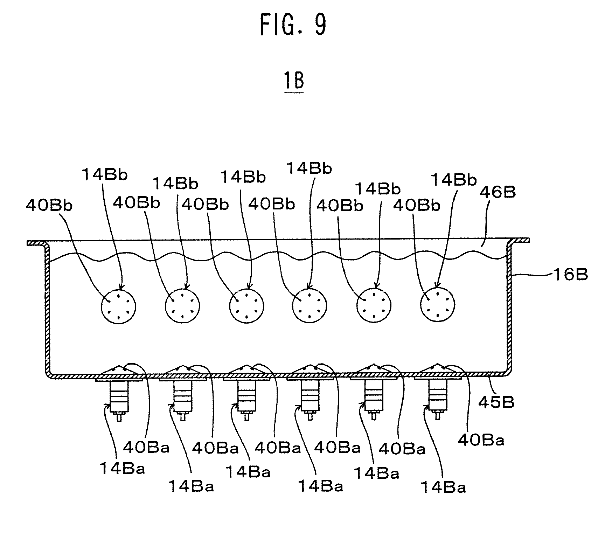

Fig. 8 is a side cross section of a cleaning tank 16B with ultrasonic transducers 14Ba and 14Bb of an ultrasonic cleaning device 1B according to a second embodiment of the present invention, and Fig. 9 is a front cross section of the ultrasonic cleaning device 1B shown in Fig. 8.

The ultrasonic cleaning device 1B according to the second embodiment includes the ultrasonic transducers 14Ba and 14Bb and the cleaning tank 16B shown in Fig. 8, and it further includes a compressor, an ozone generation device, a buffer tank and a drive portion. Since they are the same as those of the ultrasonic cleaning device 1 according to the first embodiment, so descriptions thereof will be omitted. In addition, the structure of the ultrasonic transducers 14Ba and 14Bb is the same as that of the ultrasonic cleaning device 1, so the description of the ultrasonic transducers 14Ba and 14Bb are also omitted. Note that a suffix B may be added to reference numerals in the second embodiment like the ultrasonic cleaning device 1B for distinguishing the same from the ultrasonic cleaning device 1 of the first embodiment. In addition, if the element is common to all the embodiments, the suffix B, C, D or the like of the reference numeral denoting the element may be omitted.

As shown in Figs. 8 and 9, six of the ultrasonic transducers 14Ba are attached on a bottom portion 45B of the cleaning tank 16B and six of the ultrasonic transducers 14Bb are attached on one side wall 46B (horizontal and vertical cross opposed type). The ultrasonic transducers 14Ba and 14Bb on the bottom portion 45B and on the side wall 46B are arranged so that the axis of the each transducer is positioned on a line that is perpendicular to the bottom surface in the front view as shown in Fig. 9. The ultrasonic transducers 14Ba attached to the bottom portion 45B have the same oscillation frequency, while the ultrasonic transducers 14Bb attached to the side wall 46B have the same oscillation frequency. However, the oscillation frequency of the ultrasonic transducers 14Ba attached to the bottom portion 45B is different from the oscillation frequency of the ultrasonic transducers 14Bb attached to the side wall 46B. The oscillation frequency of the ultrasonic transducers attached to one of the bottom portion 45B and the side wall 46B is set to a value two to three times higher than the oscillation frequency of the ultrasonic transducers attached to the other, and more preferably five times higher or more. For example, if the oscillation frequency of the ultrasonic transducers 14Ba attached to the bottom portion 45B is 25 kHz, the oscillation frequency of the ultrasonic transducers 14Bb attached to the side wall 46B is 150 kHz. If the oscillation frequency of the ultrasonic transducers 14Ba attached to the bottom portion 45B is 40 kHz, the oscillation frequency of the ultrasonic transducers 14Bb attached to the side wall 46B is 200 kHz.

The cleaning process performed by the ultrasonic cleaning device 1B also uses water as the cleaning fluid. The water and an object to be cleaned are put in the cleaning tank 16B. In the cleaning process by the ultrasonic cleaning device 1B, the ultrasonic transducers 14Ba and 14Bb provided to the bottom portion 45B and the side wall 46B apply ultrasonic vibrations to the water simultaneously while air containing ozone is ejected to the inside of the cleaning tank 16B.

The cleaning process by the ultrasonic cleaning device 1B is performed as follows.

Also in the ultrasonic cleaning device 1B, bubbles of the air ejected from injection holes 40Ba and 40Bb rise and disturb the liquid surface, so the depth of the liquid varies continuously. Therefore, it becomes apparently a state as if there is a sound pressure distribution of a traveling wave of the ultrasonic vibration in the cleaning tank 16B. In addition, the bubbles of the air ejected from the injection holes 40Ba and 40Bb causes a circulating flow in the cleaning tank 16B, and some of the fine bubbles easily accompany the circulating flow to reach the fine structure of the object to be cleaned. Furthermore, a contact area between the ozone and the object to be cleaned also increases.

However, in the ultrasonic cleaning device 1B, the ultrasonic transducers 14Bb are arranged on the side wall 46B adding to the ultrasonic transducers arranged on the bottom portion 45B as shown in Figs. 8 and 9. Since the oscillation frequency of the ultrasonic transducers 14Bb on the side wall 46B is different from the oscillation frequency of the ultrasonic transducers 14Ba on the bottom portion 45B, "sub harmonic interference of the traveling wave" of the ultrasonic vibration generates hydrogen peroxide in the water. The generated hydrogen peroxide reacts with the ozone to be transformed into the hydroxy radical. Thus, in the ultrasonic cleaning device 1B, the hydroxy radical having strong oxidative decomposition ability and sterilization ability is generated by the reaction between the water and the ozone as well as the reaction between the hydrogen peroxide generated in the water and the ozone. Thus, interference between the ultrasonic vibrations having different frequencies causes generation of the hydrogen peroxide so that a degree of conversion of the ozone into the hydroxy radical increases for effective cleaning and sterilization.

Also in the ultrasonic cleaning device 1B, dirt sticking to the object to be cleaned can be decomposed almost completely into carbon dioxide and water. Therefore, the waste water after the cleaning process can be discharged into the sewerage without any special waste water treatment.

### [Third embodiment]

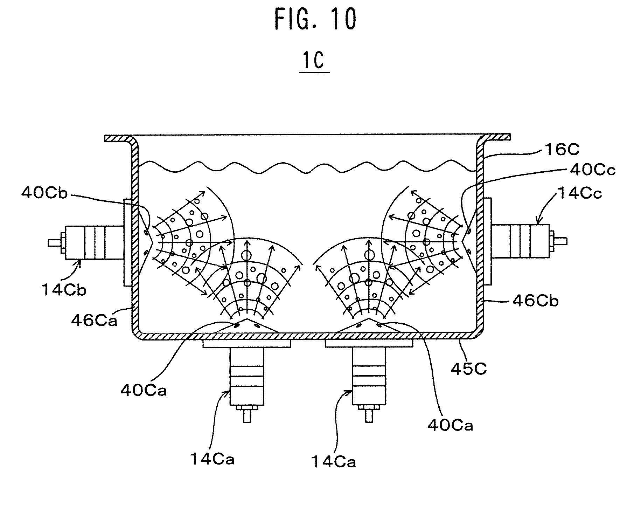

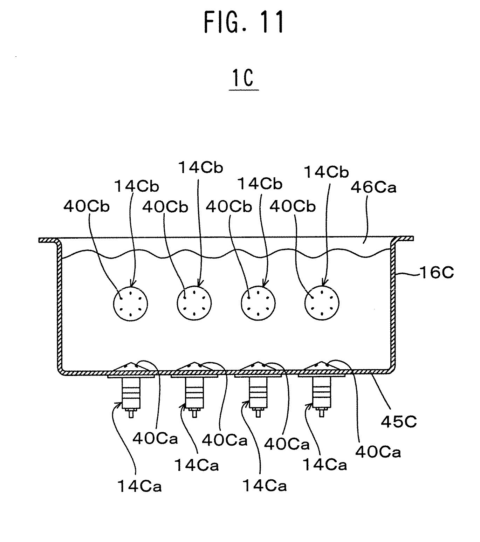

Fig. 10 is a side cross section of a cleaning tank 16C of an ultrasonic cleaning device 1C with ultrasonic transducers 14Ca-14Cc attached according to a third embodiment of the present invention, and Fig. 11 is a front cross section of the ultrasonic cleaning device 1C shown in Fig. 10.

As shown in Figs. 10 and 11, the ultrasonic transducers 14Ca-14Cc are attached on a bottom portion 45C of the cleaning tank 16C so that four transducers are aligned on each of two lines and on two opposed side walls 46Ca and 46Cb so that four transducers are aligned on one line of each side (horizontal and vertical trident interference type). The ultrasonic transducers 14Ca in two lines on the bottom portion 45C and the ultrasonic transducers 14Cb and 14Cc on the side walls 46Ca and 46Cb are arranged so that four axes of the four transducers are aligned on the plane that is perpendicular to each of the bottom portion 45C and the two side walls 46Ca and 46Cb. The eight ultrasonic transducers 14Ca attached to the bottom portion 45C have the same oscillation frequency. The eight ultrasonic transducers 14Cb and 14Cc attached to the two side walls 46Cb and 46Cc also have the same oscillation frequency. However, the oscillation frequency of the ultrasonic transducers 14Ca of the bottom portion 45C is different from the oscillation frequency of the ultrasonic transducers 14Cb and 14Cc of the side walls 46Ca and 46Cb. The oscillation frequency of the ultrasonic transducers attached to one of the bottom portion 45C and the side walls 46Ca, 46Cb is set to a value twice to three times the oscillation frequency of the ultrasonic transducers attached to the other, and more preferably five times or more.

The cleaning process is performed similarly to the case of the ultrasonic cleaning device 1B. Each of injection holes 40Ca-40Cc of the ultrasonic transducers 14Ca-14Cc ejects air containing ozone into the water inside the cleaning tank 16C, and at the same time ultrasonic vibrations are applied to the water and the object to be cleaned inside the cleaning tank 16C. The "sub harmonic interference of the traveling wave" of the ultrasonic vibration generates hydrogen peroxide in the water. As a result a lot of hydroxy radical is generated that improves the cleaning ability and the sterilization ability as described above for the case of the ultrasonic cleaning device 1B.

It is possible to perform the cleaning and sterilization process by using not the same oscillation frequency but different oscillation frequencies for the ultrasonic transducers 14Cb on one side wall 46Ca and for the ultrasonic transducer 14Cc on the other side wall 46Cb. In this case, either the oscillation frequency of the ultrasonic transducers 14Cb or that of the ultrasonic transducers 14Cc may be the same as the oscillation frequency of the ultrasonic transducers 14Ca on the bottom portion 45C.

### [Fourth embodiment]

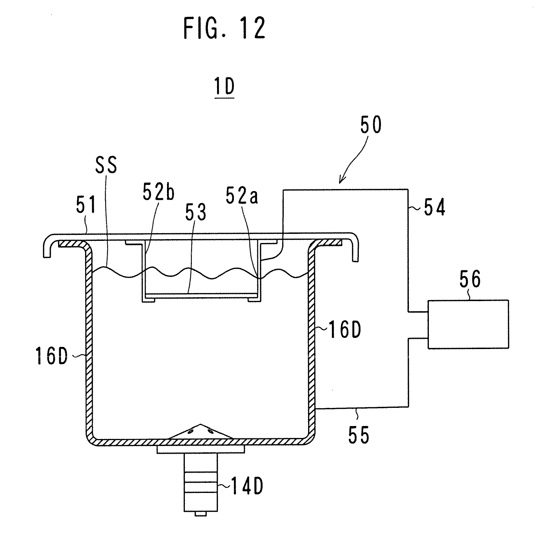

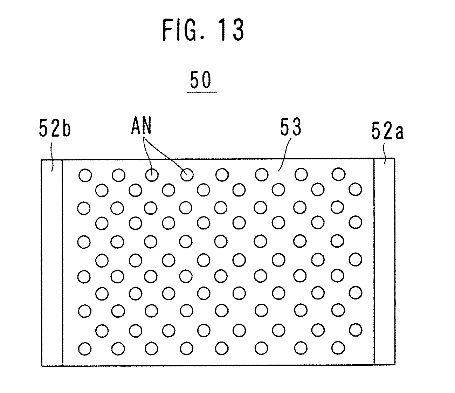

Fig. 12 is a cross section of a cleaning tank 16D of an ultrasonic cleaning device 1D according to a fourth embodiment of the present invention, and Fig. 13 is a front view of an electrode plate 53.

The fourth embodiment provides an electrolytic sterilization device 50 including the electrode plate 53 and a power supply device 56 for performing electrolytic sterilization. Other structure is the same as the first embodiment.

More specifically, as shown in Fig. 12, a lid body 51 made of an electrical insulating material such as synthetic resin is placed on the upper portion of the cleaning tank 16D at the opening portion in the fourth embodiment. Two supporting plates 52a and 52b are attached to the inside of the lid body 51. The supporting plates 52a and 52b support the electrode plate 53.

The supporting plates 52a and 52b and the electrode plate 53 are made of silver, a copper-silver alloy (e.g., copper 80% and silver 20%) or the like. They are connected electrically when the both edges of the electrode plate 53 are fixed to ends of the supporting plates 52a and 52b by screws.

As shown in Fig. 13, the electrode plate 53 is a mesh-like plate provided with a lot of circular holes so that a surface area per volume thereof is increased for improving diffusion property. The electrode plate 53 is attached to a position inside the cleaning tank 16D so as to be soaked in the cleaning fluid sufficiently.

A dc voltage V1 is applied across the electrode plate 53 and the cleaning tank 16D from the power supply device 56 via electric wires 54 and 55. The power supply device 56 is capable of outputting a dc voltage of approximately several hundred millivolts to ten and several volts. It is also capable of supplying a dc current of several milliamperes to several amperes to a load. The power supply device 56 applies the voltage so that the electrode plate 53 becomes an anode and the cleaning tank 16D becomes a cathode. Between them a current of approximately several milliamperes to several ten milliamperes is supplied. Note that it is possible to reverse the polarities of the electrodes for cleaning the cleaning tank itself and the like.

In this way, silver (Ag) ions or copper (Cu) ions are dissolved out into the cleaning fluid (tap water) by flowing a dc current between the electrode plate 53 and the cleaning tank 16D in the fourth embodiment. In the case of the copper ions, hydrogen is generated from the cathode so that the water becomes alkaline. As a result, precipitation of insoluble hydroxide is generated in the water. In the case of the silver electrode, precipitation of silver is generated on the cleaning tank surface that is the cathode, so the reaction such as the hydroxide precipitation is not considered to be a main reaction. In any case, the electrolytic sterilization can be performed by the silver ions or the copper ions. Using both the ozone sterilization and the electrolytic sterilization, almost all bacteria and virus can be sterilized so that almost perfect sterilization can be performed. In other words, according to the fourth embodiment, the sterilization action by the silver electrolytic oxidation or the sterilization action by the copper electrolytic is performed adding to the cleaning sterilization effect by the ozone described above.

Therefore, a medical instrument such as an endoscope or an operation instrument after use can be cleaned by using the ultrasonic cleaning device 1D. Thus, almost perfect cleaning, disinfection and sterilization can be performed so that other persons can use the medical instrument repeatedly and safely in a short time period.

Note that tap water contains Cl- ion at approximately 10 ppm, so current can flow in the tap water. Pure water (distilled water) scarcely permits current to flow. In the case of silver ions, the water becomes white as the electrolytic process proceeds because suspended particles of silver chloride are generated. As a result of quantitative analysis using atomic absorption method, it was found that silver ions dissolved in the tap water was approximately 0.18 ppm after removing the precipitate by a filter of 0.46 microns. This concentration is sufficient for sterilization.

It is possible to support the electrode plate 53 by an appropriate supporting member made of an insulating material so that the electrode plate 53 is soaked in the cleaning fluid instead of attaching it to the lid body 51. In addition, instead of using the cleaning tank 16D made of stainless steel as the cathode, it is possible to provide an electrode plate made of a metallic material separately as the cathode.

The above description with reference to Fig. 12 illustrates an example in which the electrolytic sterilization device 50 is added to the ultrasonic cleaning device 1 of the first embodiment. However, it is possible to structure the ultrasonic cleaning device 1D by adding the electrolytic sterilization device 50 to the ultrasonic cleaning device 1B or 1C of the second or the third embodiment or to a device of other embodiment.

Next, an experimental example of the ultrasonic cleaning device 1D and a sterilization effect thereof will be described.

### [Experimental example]

The cleaning tank 16 having an internal volume of 500 mm width, 300 mm length and 140 mm depth was used, and water of 15 liters was poured into the cleaning tank 16. Total fourteen ultrasonic transducers 14 were attached to the cleaning tank 16. More specifically, 4 x 2 of them having an oscillation frequency of 25 kHz were attached to the bottom surface, and 3 x 2 of them having an oscillation frequency of 50 kHz were attached to both side surfaces. Thus, distributed and independent vibrations were generated in a radial manner, so that the ultrasonic cleaning process is performed by an impact interference shearing action of the traveling wave accompanying the vibrations from three surfaces considering phase angles including the horizontal and the vertical interferences.

The air containing the ozone is supplied via the path 42 of the ultrasonic transducer 14. The air is ejected from the injection holes 40 to the inside of the tank as micro bubbles excited by the ultrasonic wave.

As avirulence microorganisms, colon bacilli of JM109 strain and budding yeast of FY24 strain derived from S288C strain were adopted. As the sterilization operation, four methods including only ozone, ozone and ultrasonic wave, copper electrolytic and silver electrolytic were studied. The above-mentioned colony of one strain that had been preserved in an LB plate that is a culture medium for the colon bacilli at 4°C was planted in a test tube (10, 12 or 15 milliliters) or a beaker (50 milliliters) containing culture solution on the day before. It was incubated for 12-14 hours at a temperature of 37°C to be confluent (it was bred until it cohered fully). This culture solution was put in the cleaning tank filled with the tap water of 15 liters at an appropriate ratio to be approximately 1000, 10000 and 1000000 cells per milliliter. A constant dc current was supplied to the copper electrode or the silver electrode as the anode while bubbling only the ozone, sterilizing by using both the ozone and the ultrasonic wave, or bubbling the air, so that copper ions or silver ions were melted out for the sterilization operation.

The water of 1 milliliter was sampled every two or ten minutes just after the operation was started. Then, 0.1 milliliters of the sampled water was applied and spread on the LB plate, which was incubated for 12-14 hours at 37°C. The number of colonies was measured, and a survival curve of the colon bacilli was created. The solid line in the graph showing the survival curve is an approximate curve determined by a method of moving average.

The result is as follows.

First, the cleaning effect by the ultrasonic wave like the traveling wave is as follows.

First, this device was used for examining the cleaning effect when the ultrasonic wave (intermittent mode) is operated for fifteen minutes. The ozone has strong oxidation ability and effects of sterilization, decolorization, deodorization, removal of organic matters and the like. In addition, the ozone leaves little residual because it changes back into oxygen. Simple dirt could be removed without using any detergent. Stains of sauce, soy sauce, cafe au lait, tea and mayonnaise on towel could be removed easily one hour after the stains had been made. However, stains that are left one day could not be removed only by the ozone water sufficiently. Then, a little quantity of kitchen detergent was added for removing the stains. However, the stains of tea and sauce could not be removed completely.

A bloodstain of a mouse after being dried for one day could be removed completely when the bloodstain had been made on an object made of stainless steel (such as a medical spoon)having a high mechanical impedance or an object made of plastic resin (such as a bottle with a cap made of polypropylene PP). The bloodstain made on soft gauze or a rubber glove could also be removed completely. Therefore, it was found that endoscopes and related items with bloodstains just after being used could be cleaned sufficiently without using any detergent.

The survival curves of the colon bacilli are as follows.

Concerning the colon bacilli of the JM109 strain, the ozone sterilization, the ultrasonic wave sterilization, the ultrasonic wave and ozone sterilization, the copper electrolytic sterilization and the silver electrolytic sterilization were studied. An experiment of checking the effects of sterilizing the colon bacilli was performed, and the survival curve was created for each of the effects.

The colon bacilli were perished completely by the ozone or the ozone and ultrasonic wave in twenty minutes. They were not perished only by the ultrasonic wave. The electrolytic sterilization was tried with an example of the colon bacilli so that viruses and yellow staphylococci can also be sterilized. It took 120 minutes until the colon bacilli were perished completely by using the mesh-like copper electrode. Even the dc current supplied to the copper electrode was increased from 3 mA to 20 mA, further to 40 mA, further to 60 mA and further to 1.7 A, the time necessary for sterilizing the colon bacilli could not be shortened to 60 minutes or less. The reason of this is probably that hydrogen H₂ is generated on the opposite electrode, and the remaining OH- ion reacts with Cu²⁺ to generate precipitate of Cu(OH)₂. It is probably caused when the concentration of the Cu²⁺ ion dissolved inside the electrolytic tank is too small. A large quantity of blue color precipitate of Cu(OH)₂ was deposited on the bottom portion of the electrolytic tank. When water-soluble copper sulfate CuSO₄ was dissolved, the colon bacilli were perished at once at a weight of slightly below 50 mg of the copper sulfate. It was understood that the sterilization were completed in 20 minutes by applying a dc voltage of 7.4 volts and a dc current of 60 mA to the mesh-like silver electrode. In the case of the silver electrode, it was recognized that silver is deposited on the cleaning tank as the cathode to become darkened. When silver nitrate was dissolved, the colon bacilli were perished at once at a weight of slightly below 1.5 mg of the silver nitrate. When both the ultrasonic wave ozone sterilization and the silver electrolytic sterilization were performed simultaneously in the experiment, the colon bacilli were sterilized in 10 minutes. It is noted that almost all of the colon bacilli survived a short period of time after the ozone oxidation sterilization and were perished promptly after that, but in the case of the silver electrolytic oxidation sterilization the number of dead colon bacilli increased linearly. It is probable that the former may damage a reproduction function of the colon bacilli while the latter may damage a biogenic function of the same critically.

The survival curve of the budding yeast is as follows.

Experiments of the sterilization were performed using budding yeast of the FY24 strain that is a eukaryote having a nuclear membrane and a cell wall, and the survival curve was created. It was recognized in the experiment of the sterilization using the ozone and the ultrasonic wave that the survival ratio depends on a concentration of the number of cells in the case of the yeast. More specifically, the yeast was perished at once at a concentration of 1,000 cells/milliliters (hereinafter cells/milliliters is referred to as "cell number"), and it was perished in 50 minutes at 20,000 cell number. At 40,000 cell number it was perished in 80 minutes, but at 60,000 cell number only approximately 30% of it was perished after 90 minutes. When the silver electrode was used, it was perished in 30 seconds at any of the cell numbers with a current of 60 mA supplied and in 2 minutes with a current of 10 mA. In the state where silver is deposited on the cleaning tank even a little, the budding yeast was perished promptly when it was inputted because a part of the silver was oxidized due to the air. In the experiment of using both the ultrasonic wave ozone sterilization and the silver electrolytic sterilization with a current of 60 mA, it was sterilized at once (in 30 seconds).

As shown in the results of the experiments described above, the device that was used in the experiments has a strong sterilization effect by the ultrasonic wave and the ozone cleaning as well as the ozone oxidation and the silver electrolytic oxidation. In this experiment, colon bacilli were used as a model of bacteria, and budding yeast was used as a model of fungus for evaluating the sterilization effect of this device. As a result, it is proved that this device can sterilize both the colon bacilli and the budding yeast in a short period of time. Pathogens contained in blood or other body fluid includes various types of microorganisms such as viruses, procaryote and eukaryote. It is possible to use this device for easy cleaning, disinfection and sterilization of a used endoscope to which various pathogens may be adhered.

### [Fifth embodiment]

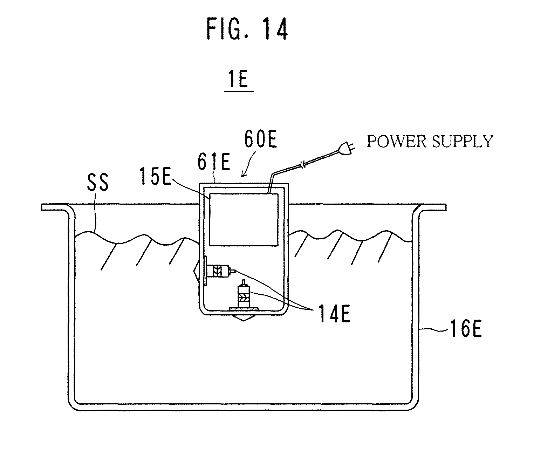

Fig. 14 is a cross section of a cleaning tank 16E of an ultrasonic cleaning device 1E according to a fifth embodiment of the present invention.

In the various embodiments described above, the ultrasonic transducers 14 are attached to the cleaning tank 16. In the fifth embodiment, however, an ultrasonic wave unit 60E to which the ultrasonic transducers 14 are attached is provided separately from the cleaning tank 16. The ultrasonic wave unit 60E is suspended above the cleaning tank 16 to be soaked in the cleaning fluid or be floated on the surface of the cleaning fluid.

More specifically, as shown in Fig. 14, the ultrasonic cleaning device 1E is made up of a cleaning tank 16E as a vessel for the cleaning fluid and the object to be cleaned, and the ultrasonic wave unit 60E.

The ultrasonic wave unit 60E includes a housing 61E having a closed structure and one or more ultrasonic transducers 14E attached to the housing 61E toward the circumference surface. A drive portion 15E is disposed inside the housing 61E, and electric wires 62 are led externally for supplying power to the drive portion 15E.

The housing 61E is formed into a box shape by using a metal plate and a synthetic resin plate or by molding synthetic resin. The ultrasonic transducers 14 described above are attached to holes provided to a wall of the housing 61E. More specifically, the oscillator 26 arranged on the inner side of the housing 61E and the vibration portion 27 arranged on the circumference surface of the housing 61E are connected by bolting. An appropriate sealing member or gasket is used for sealing the housing 61E.

The ultrasonic wave unit 60E has a sealed structure, so it floats on the surface SS of the cleaning fluid. In other words, the ultrasonic wave unit 60E becomes a floating state. In addition, if the ultrasonic transducers 14E are arranged in a compact manner inside the ultrasonic wave unit 60E, the ultrasonic wave unit 60E can be downsized. Moreover, it can be moved freely on the water surface SS or be submerged in the water. Thus, it can be moved freely in the horizontal direction and in the vertical (up and down) direction for the cleaning process. Therefore, the shape, the dimensions and the depth of the cleaning tank 16E can be selected freely.

In addition, since the housing 61E is made of a metallic material to have a sealed structure, the drive portion 15E inside the housing 61E can be shielded in an electromagnetic manner so that unwanted emission noise is suppressed. However, it is possible to provide the drive portion 15E outside the housing 61E instead of inside the same.

An example of a procedure of the cleaning process is as follows. First, the cleaning tank 16E is filled with the cleaning fluid, and the object to be cleaned is put in the cleaning fluid. In this case, it is possible to use a mesh-like basket that can be inserted in the cleaning tank 16E and to put the object to be cleaned in the mesh-like basket, which is put in or out of the cleaning tank 16E. Then, the ultrasonic wave unit 60E is floated on the water surface SS. The ultrasonic wave unit 60E is moved in the cross direction and in the lateral direction while the ultrasonic transducer 14E is driven. In addition, the ultrasonic wave unit 60E is submerged in the water. When the cleaning process is finished, the ultrasonic wave unit 60E is taken out and then the object to be cleaned is taken out.

Note that the housing 61E is not always required to have a sealed structure. For example, there may be a certain gap or opening on the upper portion of the housing 61E if it is arranged at a position having a height such that the cleaning fluid does not enter the inside of the housing 61E.

Next, variations of the structure in the fifth embodiment will be described.

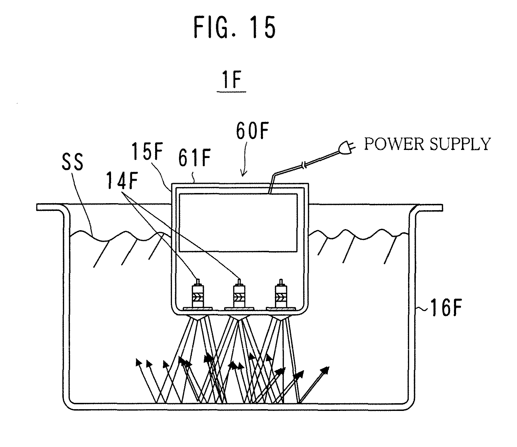

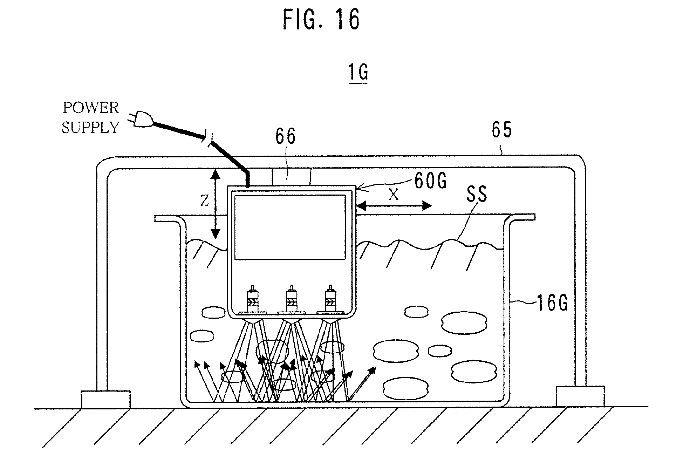

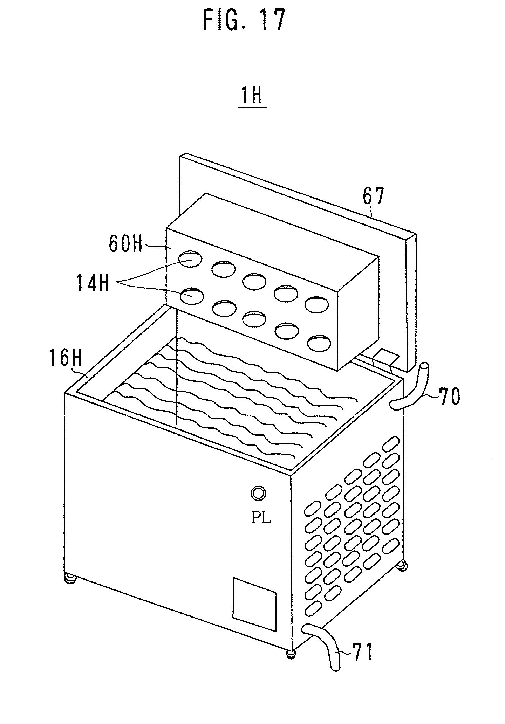

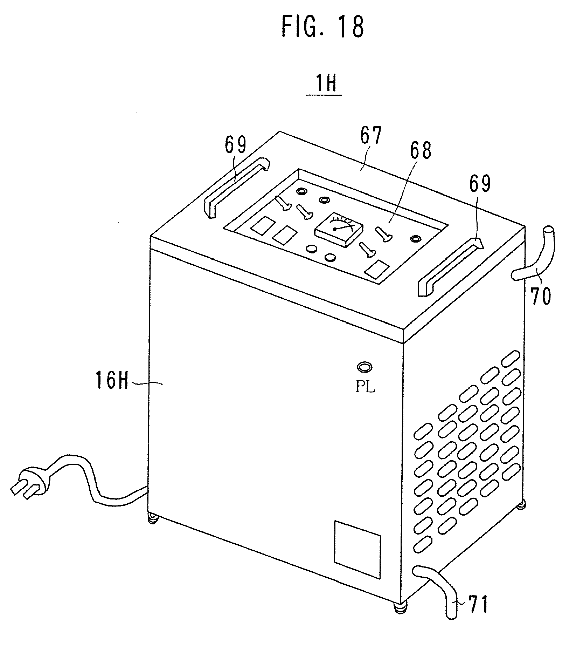

Fig. 15 is a cross section of an ultrasonic cleaning device 1F according to a first variation, Fig. 16 is a cross section of an ultrasonic cleaning device 1G according to a second variation, Fig. 17 is a perspective view of an ultrasonic cleaning device 1H with a lid opened according to a third variation, and Fig. 18 is a perspective view of the ultrasonic cleaning device 1H with the lid closed according to the third variation.

The ultrasonic cleaning device 1F shown in Fig. 15 includes an ultrasonic wave unit 60F having ultrasonic transducers 14F all of which are attached to the bottom surface of a housing 61F in the state of facing downward. Therefore, the object to be cleaned that is placed at the vicinity of the bottom surface of the cleaning tank 16F can be cleaned evenly and efficiently.

The ultrasonic cleaning device 1G shown in Fig. 16 includes a suspension beam 65 that is supported in the horizontal direction by legs standing on the floor. An ultrasonic wave unit 60G is suspended by an arm 66 from the suspension beam 65 and can be driven to move in the horizontal direction along the suspension beam 65. In other words, the suspension beam 65, the arm 66 and the like constitute a device for moving the unit.

Therefore, the ultrasonic wave unit 60G is driven to move in the horizontal direction, so that the object to be cleaned inside a cleaning tank 16G can be cleaned evenly and efficiently.

It is more preferable that the ultrasonic wave unit 60G can be driven to move in both the X direction and the Y direction that are the horizontal directions. It is also preferable that the ultrasonic wave unit 60G can be driven to move in the Z direction that is the vertical direction. In addition, it is possible to set the height of the suspension beam 65 high enough for the ultrasonic wave unit 60G to move in the vertical direction. Thus, after the ultrasonic wave unit 60G is moved upward, the cleaning tank 16G can be moved in the horizontal direction for exchanging the cleaning tank 16G. In this structure, a lot of cleaning tanks 16G are moved in the horizontal direction by a conveyer for example, so that the cleaning process can be performed continuously at the position of the ultrasonic wave unit 60G. In addition, a plurality of ultrasonic wave units 60G may be arranged so that preparatory cleaning, main cleaning, rinsing and the like of clothes for example can be performed sequentially in one cleaning tank 16G.

The ultrasonic cleaning device 1H shown in Figs. 17 and 18 has a cleaning tank 16H that is also the entire housing, and a lid body 67 is attached to the upper portion of the cleaning tank 16H in an openable and closable manner. An ultrasonic wave unit 60H that is similar to the one described above is attached to the back surface of the lid body 67. A control panel 68 and two handles 69 and 69 are attached to the surface of the lid body 67. In addition, a water supply inlet 70 is provided to the upper portion on the side surface of the cleaning tank 16H, and a waste water outlet 71 is provided to the lower portion on the same.

According to the ultrasonic cleaning device 1H, the object to be cleaned can be cleaned easily by the following steps. The steps include putting an object to be cleaned in the cleaning tank 16H after opening the lid body 67, supplying cleaning fluid through the water supply inlet 70, closing the lid body 67 and operating the control panel 68 for turning on the power supply. In addition, it is easy to install the ultrasonic cleaning device 1H and to move the same.

It is also possible to combine various features of the ultrasonic cleaning devices 1 and 1B-1D with the ultrasonic cleaning devices 1E-1H in the fifth embodiment and its variations.

As described above, according to the present embodiment, it is possible to provide the ultrasonic cleaning method and device that can perform simultaneously the cleaning process and the sterilizing process of the medical appliances or the like. The ultrasonic cleaning method and device can also be used for washing hands or the like for disinfection, and waste water thereof can be discharged without any special waste water treatment.

In the various embodiments described above, various types of pumps can be used for the compressor 11. If a rotary compressor is used, the buffer tank 13 can be omitted.

The shape of the head portion of the vibration portion provided to the ultrasonic transducer can be a pyramidal shape or a curved surface that is a part of a spherical surface other than the conical shape. If the head portion has such a shape having a surface inclined with respect to the axis, the ultrasonic vibrations that are radiated from the vibration portion into the water are radiated not only in the axial direction of the ultrasonic transducer but also in other directions, so that ultrasonic vibrations radiated from neighboring vibration portions are interfered with each other. Therefore, even in the case where the ultrasonic transducers 14 are attached only to the bottom portion 45 as shown in Fig. 7, interferences of the ultrasonic vibrations are generated so that the effect of the interferences can be obtained. Further, if neighboring ultrasonic transducers 14 generate ultrasonic vibrations of different frequencies, more effective interference of the ultrasonic vibrations is generated. As a result, generation of the hydroxy radical is promoted, and the effect of the cleaning and the sterilization can be enhanced.

The air containing the ozone may be ejected into the water from a discharging outlet of an air supplying portion that is provided separately from the ultrasonic transducer instead of being ejected from the injection hole of the ultrasonic transducer. In this case, it is preferable to dispose the discharging outlet just above the vibration portion or at the vicinity thereof for generating micro bubbles efficiently.

In addition, it is possible to change the arrangement of the ultrasonic transducers in Figs. 7-11. For example, in Fig. 7, they may be arranged like a 4 + 3 triangle in which four aligned transducers and three aligned transducers are arranged in an alternating manner instead of 4 × 2 rectangular arrangement. In addition, it is possible to modify the above-mentioned arrangement of the set of ultrasonic transducers 14Ba and 14Bb attached to the bottom portion 45B and the side wall 46B as shown in Fig. 8 as well as the arrangement of the set of ultrasonic transducers 14Ca-14Cc attached to the bottom portion 45C and the side walls 46Ca and 46Cb as shown in Fig. 10. For example, the ultrasonic transducers 14Ca-14Cc can be arranged in a zigzag pattern in a plan view. The plurality of ultrasonic transducers attached to the bottom portion or the same side wall may have different oscillation frequencies.

The ultrasonic cleaning device according to the present embodiment can work effectively for the cleaning method with the disinfection and the sterilization. In addition, the cleaning device is always disinfected and sterilized by itself, so it is not necessary to perform precleaning, disinfection and sterilization before the cleaning process unlike the conventional device. Therefore, an excessive consumption of water can be avoided as much as possible, and other advantages can be obtained in various cleaning processes.

In the embodiments described above, it is possible to make the cleaning tank by using resin or as a film-like vessel. It is also possible to make the cleaning tank as a folding one. In this case, a portable ultrasonic cleaning device can be realized with ultrasonic transducers and a nozzle for ejecting air containing ozone that are set as necessity.

Other than that, the structure, the shape, the dimensions, the number of elements, the arrangement and the positions of the whole or a part of the compressor, the ozone generation device, the buffer tank, the ultrasonic transducer, the drive portion, the cleaning tank, the pedestal, the electrolytic sterilization device, the electrode plate, the power supply device, the ultrasonic wave unit and its supporting device or the ultrasonic cleaning device can be modified if necessary in accordance with the spirit of the present invention.

### INDUSTRIAL APPLICABILITY

The present invention is usable for cleaning, disinfecting and sterilizing not only commodities and valuables such as glasses or jewelry but also other various objects to be cleaned including medical appliances such as forceps that are used for an operation or a therapy, fabrics such as clothes or gauze and a soft object such as leather.

As another epoch-making application, it can be used for cleaning vegetables and fruits as well as cut vegetables these days and vessels for school feeding.

## Claims

1. An ultrasonic cleaning method for cleaning an object to be cleaned by using an ultrasonic wave, the method comprising the steps of:
putting cleaning fluid and the object to be cleaned in a cleaning tank; and
applying ultrasonic vibrations to the cleaning fluid from a vibration portion that is disposed inside the cleaning tank for generating ultrasonic vibrations while ejecting air containing ozone into the cleaning fluid.

2. An ultrasonic cleaning method for cleaning an object to be cleaned by using an ultrasonic wave, the method comprising the steps of:
putting cleaning fluid and the object to be cleaned in a cleaning tank; and
applying ultrasonic vibrations to the cleaning fluid from two or more vibration portions having different directions of radiating the ultrasonic wave simultaneously while ejecting air containing ozone into the cleaning fluid, the vibration portions being disposed inside the cleaning tank.

3. An ultrasonic cleaning device comprising:
a cleaning tank;
an ultrasonic vibration member having a structure in which an ultrasonic oscillator and a vibration portion disposed inside the cleaning tank are connected to each other;
an ozone generation device for generating ozone from air; and
an ozone supplying portion that transfers the air containing the ozone generated by the ozone generation device so that the air containing the ozone is discharged into the cleaning tank.

4. An ultrasonic cleaning device comprising:
a cleaning tank;
an ultrasonic vibration member having a structure in which an ultrasonic oscillator and a vibration portion disposed inside the cleaning tank are connected to each other, the vibration portion having a path inside that opens to the inside of the cleaning tank;
an ozone generation device for generating ozone from air; and
an ozone supplying portion that supplies the air containing the ozone generated by the ozone generation device to the path.

5. The ultrasonic cleaning device according to claim 4, comprising a plurality of the ultrasonic vibration members, wherein
some or all of the vibration portions of the ultrasonic vibration members have an end portion of a conical, a pyramidal or a curved surface, and
some or all of the vibration portions of the ultrasonic vibration members are attached to the cleaning tank directly or indirectly via a shock absorbing member.

6. The ultrasonic cleaning device according to claim 5, wherein the plurality of ultrasonic vibration members are attached to the cleaning tank so that oscillation directions of the ultrasonic oscillators become two or more different directions.

7. The ultrasonic cleaning device according to claim 5 or 6, wherein the plurality of ultrasonic vibration members are driven electrically at an output power of 50 watts or less so as to generate ultrasonic vibration independently from each other.

8. The ultrasonic cleaning device according to any one of claims 3 to 7, wherein a drive circuit for driving the cleaning tank, the ozone generation device, the ozone supplying portion and the ultrasonic vibration member is integrated to a pedestal, the ozone supplying portion includes an air reservoir for holding air containing ozone, and the air reservoir is disposed at a position higher than a surface of cleaning fluid in the cleaning tank.

9. The ultrasonic cleaning method according to claim 1 or 2, wherein the vibration portion has a path that opens at the surface thereof, and the air containing the ozone is discharged into the cleaning fluid from the path.

10. The ultrasonic cleaning method according to claim 1 or 2, wherein a silver electrode is disposed in the cleaning fluid so that silver ions are melted out, and electrolytic sterilization by the silver ions is performed for the object to be cleaned adding to the sterilization by the ozone.

11. The ultrasonic cleaning method according to claim 1 or 2, wherein a copper electrode is disposed in the cleaning fluid so that copper ions are melted out, and electrolytic sterilization by the copper ions is performed for the object to be cleaned adding to the sterilization by the ozone.

12. The ultrasonic cleaning device according to claim 3 or 4, further comprising two electrode plates disposed to be soaked in the cleaning fluid and a power supply device for applying a voltage across the two electrode plates, wherein the ultrasonic cleaning device is structured to perform electrolytic sterilization for the object to be cleaned.

13. The ultrasonic cleaning device according to claim 12, wherein one of the electrode plates is a silver electrode plate, and the electrolytic sterilization is silver electrolytic sterilization by using the silver electrode plate.

14. The ultrasonic cleaning device according to claim 3 or 4, wherein the cleaning tank is made of a metal material, an electrode plate containing silver is disposed to be soaked in the cleaning fluid without contacting the cleaning tank electrically at the upper portion of the cleaning tank, a power supply device is provided for applying a voltage across the electrode plate and the cleaning tank, and the ultrasonic cleaning device is structured to perform silver electrolytic sterilization for the object to be cleaned.

15. An ultrasonic cleaning device comprising:
a cleaning tank;
an ultrasonic wave unit that is structured to include a housing and an ultrasonic vibration member having an ultrasonic oscillator disposed inside the housing and a vibration portion disposed on the circumference surface of the housing, the ultrasonic oscillator and the vibration portion being connected to each other, and the ultrasonic wave unit being soaked in the cleaning fluid inside the cleaning tank;
an ozone generation device for generating ozone from air; and
an ozone supplying portion that transfers the air containing the ozone generated by the ozone generation device and discharges the same into the cleaning tank.

16. The ultrasonic cleaning device according to claim 15, wherein the vibration portion has a path that opens at a surface thereof, and the air containing the ozone is transferred to the path and is discharged into the cleaning fluid from the path.

17. The ultrasonic cleaning device according to claim 15 or 16, wherein the housing has a sealed structure so that the housing is floated on a surface of the cleaning fluid.

18. The ultrasonic cleaning device according to any one of claims 15 to 17, further comprising a unit moving device for moving the ultrasonic wave unit above the cleaning tank.
